# EUROPÄISCHE PATENTSCHRIFT

(11) **EP 0 392 154 B1**
(45) Veröffentlichungstag und Bekanntmachung des Hinweises auf die Patenterteilung: **02.11.1994**
(21) Anmeldenummer: 90102699.7
(22) Anmeldetag: 12.02.1990
(51) Int. Cl.: G01N 1/24, G01N 33/00

(54) **Vorrichtung zur Messung von Schwefeldioxyd (SO2) mit einem Gasanalysengerät**
Device for measuring the sulphur dioxide (SO2) with a gas analysing apparatus
Dispositif de mesure du dioxyde de soufre (SO2) avec un appareil d'analyse de gaz

(30) Priorität: 12.04.1989 DE 8904607 U
(43) Veröffentlichungstag der Anmeldung: 17.10.1990
(73) Patentinhaber: Hartmann & Braun Aktiengesellschaft, 60484 Frankfurt am Main (DE)
(72) Erfinder: Ruse, Alios, D-6370 Oberursel 6 (DE)
(74) Vertreter: Presting, Hans-Joachim, Dipl.-Ing.

(56) Entgegenhaltungen:
- EP-A- 0 194 955
- DE-A- 2 216 179
- DE-U- 8 904 607
- STAUB-REINHALTUNG DER LUFT Band 45, Nr. 9, September 1985, Seiten 414-418, Düsseldorf, DE; J. DIVISEK et al.: "Kontinuierliches SO2-Messgerät für die zuverlässige Überwachung von Feuerungsabgasen"
- SIEMENS POWER ENGINEERING Band 5, Nr. 2, März-April 1983, Seiten 52-55, Passau, DE; H.J. SEIBERTH: "Recorded Measurement of Sulphur Dioxide Emissions from Utilities"

## Beschreibung

Die Erfindung bezieht sich auf eine Vorrichtung nach dem Oberbegriff des Anspruchs 1.

Eine Vorrichtung der gattungsgemäßen Art ist aus dem Aufsatz "Kontinuierliches SO₂-Meßgerät für die zuverlässige Überwachung von Feuerungsabgasen" (Staub, Reinhaltung der Luft, Band 45 (1985), Nr. 9 - September) bekannt. Hierbei wird das Meßgas über eine Gasentnahmesonde aus dem Abgaskamin einer Feuerungsanlage entnommen. Das Meßgas wird über eine Meßleitung und einen Meßgaskühler letztlich dem Meßgasanalysator zugeführt. Hierbei entsteht das generelle Problem, daß beim Führen des Meßgases über den Meßgaskühler das eigentlich zu messende Schwefeldioxyd teilweise über das Kondensat abgeführt wird und die so fehlende Meßgasmenge zu einer Fehlbewertung des Brennzustandes der Feuerungsanlage führt. Dieses Problem ergibt sich aus folgendem Grund.

Bei der Messung von Schwefeldioxyd (SO₂) in Rauchgas wird das Meßgas über einen Gaskühler geführt, der das Gas auf einige Grad Celsius abkühlt. Dabei fällt Wasser als Kondensat aus. Bei der Kondensation des Wasserdampfes löst sich unter anderen auch ein gewisser Anteil des Schwefeldioxyds im Kondensat und verursacht damit Meßfehler. Bei SO₂-Konzentrationen von 1000 mg SO₂/m³ und größer werden die SO₂-Verluste im Gaskühler bei der Messung pauschal berücksichtigt, da hier der Meßfehler nur wenige Prozente beträgt. Bei SO₂-Konzentrationen kleiner 500 mg/m³ steigen die SO₂-Verluste, bezogen auf den Meßwert, jedoch so stark an, daß eine pauschale Meßwertkorrektur nicht mehr in Frage kommt.

Der Erfindung liegt daher die Aufgabe zugrunde, eine Einrichtung zur Verminderung von SO₂-Verlusten bei der Kondensation von Wasserdampf in der Meßgasaufbereitung einer SO₂- Analyseneinrichtung anzugeben.

Die Lösung dieser Aufgabe gelingt mit den im Kennzeichen des Anspruches 1 angegebenen Mitteln. Vorteilhafte Ausgestaltungen der Erfindung sind in den Unteransprüchen angegeben.

Mit der Zuführung dosierter Mengen einer Säure oder einer wässrigen Lösung eines Salzes in den SO₂-haltigen Gasstrom an der Stelle in der Meßleitung, an der Wasserdampf kondensiert, wird der pH-Wert des Kondensates soweit herabgesetzt, daß sich die Löslichkeit für Schwefeldioxyd verringert. Damit wird der Löslichkeitsfehler reproduzierbar und er ist in die Konzentrationsmessung für Schwefeldioxyd eineichbar. Der pH-Wert des aus dem Meßgaskühler ausfließenden Kondensat-Säure-Gemisches sollte unter 1,5 liegen. Anderenfalls ist die Konzentration der verwendeten Säure oder die dosierte Säuremenge zu erhöhen. Bei der Messung von SO₂ im Rauchgas von Zementwerken haben sich 5 %ige Lösungen von Phosphorsäure (H3PO4) oder Schwefelsäure (H2SO4) ebenso bewährt wie wässrige Lösungen saurer Salze, z.B. von Natrium- oder Kaliumhydrogensulfat (NaHSO4). Auch zum Auswaschen alkalischer Gase, wie Ammoniak (NH3) oder Ammonchlorid (NH4Cl), aus Rauchgasen hat sich die Methode bewährt.

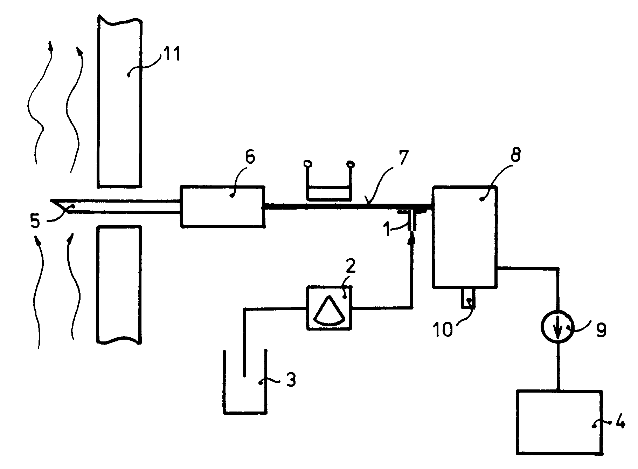

Die Erfindung wird anhand eines in der Figur dargestellten Ausführungsbeispieles näher erläutert.

Die Figur zeigt eine Vorrichtung zur Messung von Schwefeldioxyd-Emissionen in einem Rauchgaskanal 11, beispielsweise eines Zementwerkes. Über eine Gasentnahmesonde 5 mit einem Filter 6 wird eine Meßgasprobe entnommen, in einem Meßgaskühler 8 aufbereitet und durch eine Membranpumpe 9 einem Gasanalysengerät 4 (z.B. ein IR-Gasanalysator) zugeleitet. Am Ende der beheizten Meßgasleitung 7 ist vor dem Meßgaskühler 8 ein Zulauf 1 angeordnet, über den mittels einer Schlauchpumpe 2 in vorgegebenen zeitlichen Abständen aus einem Vorratsgefäß 3 dosierte Mengen einer Säure oder einer wässrigen Lösung eines sauren Salzes in den Meßgasstrom eingeleitet werden. Je nach Länge der Säureförderschläuche beginnt die Säure nach einiger Zeit dem Gasstrom zuzufließen und benetzt die Innenwandung der Meßgasleitung 7.

Der pH-Wert des aus dem Gaskühler 8 ausfließenden Kondensat-Säure-Gemisches sollte unter einem Wert von 1,5 liegen. Die Säuredosierung erfolgt am Ende der beheizten Meßgasleitung 7. Unmittelbar danach durchströmt das Meßgas mit dem Säuerungsmittel den Gaskühler 8, in dem die Säure und das Kondensat vom Gasstrom abgetrennt und über den Kondensatablaß 10 ausgetragen wird.

Zum Auswaschen von Ammoniak oder von Ammoniumsalzen aus dem Meßgas werden der Zulauf 1 und der Meßgaskühler 8 unmittelbar an das Filter 6 angeschlossen. Dadurch wird die nachfolgende beheizte Meßgasleitung 7 vor sublimierenden NH4-Salzen geschützt.

## Patentansprüche

1. Vorrichtung zur Messung von Schwefeldioxid enthaltend ein Gasanalysegerät (4), welchem über eine Meßleitung (7) und einen Meßgaskühler (8) ein über eine Gasentnahmesonde (5) entnommenes Meßgas zugeführt wird,
dadurch gekennzeichnet,
daß die Meßleitung (7) vor Eintritt in den Meßgaskühler (8) einen Zulauf (1) enthält und Mittel (2,3) vorhanden sind, die über den Zulauf (1) dosierbare Mengen einer Säure oder einer wässrigen Lösung eines sauren Salzes in den Meßgasstrom einleiten.

2. Vorrichtung zur Messung von Schwefeldioxid nach Anspruch 1,
dadurch gekennzeichnet,
daß der Zulauf (1) über ein Vorratsgefäß speisbar ist, welches die Säure oder die wässrige Lösung eines sauren Salzes enthält.

3. Vorrichtung zur Messung von Schwefeldioxid nach Anspruch 2,
dadurch gekennzeichnet,
daß an den Zulauf (1) eine Schlauchpumpe (2) angeschlossen ist.

4. Vorrichtung zur Messung von Schwefeldioxid nach den Ansprüchen 1 - 3,
dadurch gekennzeichnet,
daß 5 %-ige Phosphorsäure (H₃PO₄) oder 5 %-ige Schwefelsäure (H₂SO₄) verwendet wird.

5. Vorrichtung zur Messung von Schwefeldioxid nach den Ansprüchen 1 - 3,
dadurch gekennzeichnet,
daß 10 %-iges Natriumhydrogensulfat (NaHSO₄) oder 10 %-iges Kaliumhydrogensulfat (KHSO₄) verwendet wird.

6. Vorrichtung zur Messung von Schwefeldioxid nach einem oder mehreren der vorhergehenden Ansprüche,
dadurch gekennzeichnet,
daß die Meßleitung (7) beheizbar ist.

## Claims

1. An apparatus for measuring sulphur dioxide, comprising a gas analysis apparatus (4), to which a measuring gas sampled by means of a gas sampling probe (5) is fed via a measuring line (7) and a measuring gas cooler (8), characterised in that the measuring line (7) before entry into the measuring gas cooler (8) contains an inlet (1) and means (2, 3) are present which introduce meterable quantities of an acid or of an aqueous solution of an acid salt into the measuring gas stream via the inlet (1).

2. An apparatus for measuring sulphur dioxide according to Claim 1, characterised in that the inlet (1) can be fed via a storage vessel which contains the acid or the aqueous solution of an acid salt.

3. An apparatus for measuring sulphur dioxide according to Claim 2, characterised in that a hose pump (2) is connected to the inlet (1).

4. An apparatus for measuring sulphur dioxide according to Claims 1 to 3, characterised in that 5%-strength phosphoric acid (H₃PO₄) or 5%-strength sulphuric acid (H₂SO₄) is used.

5. An apparatus for measuring sulphur dioxide according to Claims 1 to 3, characterised in that 10%-strength sodium hydrogen sulphate (NaHSO₄) or 10%-strength potassium hydrogen sulphate (KHSO₄) is used.

6. An apparatus for measuring sulphur dioxide according to one or more of the preceding Claims, characterised in that the measuring line (7) is heatable.

## Revendications

1. Dispositif pour mesurer le dioxyde de soufre, comportant un appareil d'analyse de gaz (4) auquel est amené, par l'intermédiaire d'un conduit de mesure (7) et d'un refroidisseur de gaz de mesure (8), un gaz de mesure extrait par l'intermédiaire d'une sonde d'extraction de gaz (5),
caractérisé en ce que le conduit de mesure (7), avant l'entrée dans le refroidisseur de gaz de mesure (8), comporte une arrivée (1), et des moyens (2,3) sont prévus, qui, par l'intermédiaire de l'arrivée (1), introduisent des quantités dosables d'un acide ou d'une solution aqueuse d'un sel d'acide dans le courant de gaz de mesure.

2. Dispositif pour mesurer le dioxyde de soufre selon la revendication 1,
caractérisé en ce que l'arrivée (1) peut être alimentée par l'intermédiaire d'une réserve, laquelle contient l'acide ou la solution aqueuse d'un sel d'acide.

3. Dispositif pour mesurer le dioxyde de soufre selon la revendication 2,
caractérisé en ce qu'une pompe tubulaire (2) est raccordée à l'arrivée (1).

4. Dispositif pour mesurer le dioxyde de soufre selon les revendications 1 à 3,
caractérisé en ce que de l'acide phosphorique à 5% (H₃PO₄) ou de l'acide sulfurique à 5% (H₂SO₄) est utilisé.

5. Dispositif pour mesurer le dioxyde de soufre selon les revendications 1 à 3,
caractérisé en ce que de l'hydrogénosulfate de sodium à 10% (NaHSO₄) ou de l'hydrogénosulfate de potassium à 10% (KHSO₄) est utilisé.

6. Dispositif pour mesurer le dioxyde de soufre selon une ou plusieurs des revendications précédentes,
caractérisé en ce que le conduit de mesure (7) peut être chauffé.
